# FASCICULE DE BREVET EUROPEEN

(11) **EP 0 657 386 B1**
(45) Date de publication et mention de la délivrance du brevet: **10.06.1998**
(21) Numéro de dépôt: 94402834.9
(22) Date de dépôt: 09.12.1994
(51) Int. Cl.: C01G 55/00, B01J 23/46, C07C 51/12

(54) **Procédé de préparation d'une solution à base d'iridium, solution obtenue et utilisation de celle-ci en tant que catalyseur**
Verfahren zur Herstellung einer Lösung auf der Basis von Iridium, erhaltene Lösung und seine Verwendung als Katalysator
Process for the preparation of an iridium-containing solution, the solution obtained and its use as catalyst

(30) Priorité: 10.12.1993 FR 9314844
(43) Date de publication de la demande: 14.06.1995
(73) Titulaire: ACETEX CHIMIE, 92082 Paris La Défense 2 (FR)
(72) Inventeur: Nobel, Dominique, F-69270 Fontaines Saint Martin (FR)
(74) Mandataire: Giraud, Françoise

(56) Documents cités:
- EP-A- 0 092 350
- ZEITSCHRIFT FUR ANORGANISCHE UND ALLGEMEINE CHEMIE, vol.147, 1925, LEIPZIG DD pages 265 - 287 F. KRAUSS, H. GERLACH 'Über die Halogenide des Iridiums'
- CHEMICAL ABSTRACTS, vol. 77, no. 16, 16 Octobre 1972, Columbus, Ohio, US; abstract no. 108726v, STYRKAS 'DISSOLUTION OF PLATINUM METALS' page 510 ;

## Description

La présente invention a trait à la préparation d'une solution à base d'iridium, à la solution ainsi obtenue ainsi qu'à l'utilisation de celle-ci en tant que catalyseur.

L'iridium est un catalyseur bien connu et utilisé dans de nombreux types de réactions. On peut citer à titre d'exemple, l'utilisation de systèmes catalytiques à base d'iridium dans des réactions de carbonylation de composés du type des alcools, éthers, esters d'acides carboxyliques, dans le but de fabriquer notamment, les acides carboxyliques ou anhydrides d'acides carboxyliques. On connaît aussi des applications de l'iridium en tant que catalyseur dans des réactions d'hydroformylation d'oléfines pour obtenir des aldéhydes. Ce type de catalyseur peut de même être utilisé pour l'obtention d'acide acétique par isomérisation du formiate de méthyle, ou peut encore être employé dans la réaction de gaz à l'eau.

Dans le cas plus particulier de l'obtention d'acides carboxyliques par carbonylation, le brevet américain US 3 772 380 décrit l'utilisation d'un système catalytique, se présentant sous une forme soluble ou non dans le milieu réactionnel. Ce système catalytique est à base d'iridium, pouvant être associé à un halogène, et à base d'un halogénure covalent, tel qu'un halogénure d'alkyle par exemple. L'iridium peut être introduit dans le mélange réactionnel soit directement sous la forme d'un composé comprenant l'halogène, soit sous la forme de deux composés distincts en tant que précurseurs du composé final comprenant l'iridium et l'halogène.

On constate cependant l'existence de nombreux inconvénients à procéder, à l'échelle industrielle, comme décrit dans ce brevet, plus particulièrement dans le cadre d'une catalyse réalisée en phase homogène. En effet, les composés mentionnés dans ledit brevet ne sont pas, pour la plupart, disponibles industriellement et leur synthèse est très complexe et onéreuse. Par ailleurs, ces composés peuvent contenir des éléments étrangers au système qui risquent de polluer le milieu réactionnel ou encore de perturber la conduite de la réaction.

Il est aussi mentionné dans ce brevet, la possibilité d'utiliser des composés solides et de les mettre directement en contact avec le milieu réactionnel. Cependant, la pratique industrielle n'est pas conforme à un tel mode opératoire. En effet, cela implique une période d'initiation de la réaction (donc de moindre production), correspondant au temps nécessaire à l'obtention du catalyseur final soluble. Cela peut aussi être la cause d'une inhibition du catalyseur ou encore d'une augmentation de la production de sous produits.

Enfin, pour ce qui a plus particulièrement trait aux composés comprenant de l'iridium et de l'oxygène, ce brevet ne décrit pas de conditions opératoires dans lesquelles lesdits composés peuvent être solubilisés pour donner une solution catalytique homogène au milieu réactionnel.

La présente invention a pour but de pallier les divers inconvénients mentionnés ci-dessus. Ainsi elle décrit une voie d'accès à une solution comprenant de l'iridium, exploitable à l'échelle industrielle, c'est-à-dire ne nécessitant pas la mise en oeuvre d'une série d'étapes complexes et/ou coûteuses ni l'utilisation de réactifs non disponibles sur le marché.

L'invention permet en outre de résoudre le problème de la mise en solution de composés comprenant de l'iridium et de l'oxygène, d'une part, et leur transformation en un composé directement actif, d'autre part.

En effet, il est bien connu que ce type de composés est très difficilement soluble, dans les acides notamment, voire insoluble dans des mélanges tels que l'eau régale, comme c'est le cas pour l'oxyde d'iridium (IV) anhydre.

Enfin, l'invention permet d'obtenir une solution utilisable directement en tant que catalyseur, au moins aussi efficace que les catalyseurs connus dans des conditions identiques, et ne présentant pas, en outre, de période d'initiation lors de leur utilisation.

Ainsi, la présente invention a pour objet un procédé de préparation d'une solution à base d'iridium dans lequel on met en contact, en phase liquide, au moins un oxyde ou un hydroxyde, hydraté ou non, d'iridium, en présence d'un solvant, avec de l'acide iodhydrique ou un composé susceptible de libérer de l'acide iodhydrique, dans une quantité telle que le nombre de moles d'acide iodhydrique rapporté au nombre de moles d'iridium soit compris entre 1 et 100.

La présente invention concerne en outre une solution à base d'iridium susceptible d'être obtenue en mettant en oeuvre ledit procédé.

Enfin, un autre objet de l'invention a trait à l'utilisation des solutions à base d'iridium mentionnées ci-dessus en tant que catalyseur.

Mais d'autres avantages et caractéristiques de la présente invention apparaîtront plus clairement à la lecture de la description et des exemples qui vont suivre.

Ainsi que cela a été indiqué plus haut, le procédé selon l'invention consiste à mettre en contact en phase liquide un composé de l'iridium avec de l'acide iodhydrique ou un composé susceptible de libérer de l'acide iodhydrique, ledit composé étant choisi parmi les oxydes ou hydroxydes, hydratés ou non, d'iridium, seuls ou en mélange.

Plus particulièrement le degré d'oxydation de l'iridium dans ces composés varie entre (III) et (IV).

Les composés suivants conviennent à la mise en oeuvre du procédé selon l'invention : Ir₂O₃, xH₂O ; IrO₂, yH₂O ; formules dans lesquelles x est compris entre 0 et 10 et y entre 0 et 5.

Bien entendu, les composés à base d'iridium convenables pour la mise en oeuvre de l'invention ne sont pas limités à cette seule liste, le principal intérêt de celle-ci étant dû au fait que les composés qu'elle regroupe sont disponibles sur le marché. Par conséquent tous les composés iridiés comprenant essentiellement de l'oxygène, éventuellement des molécules d'eau conviennent au procédé.

Les oxydes ou hydroxydes d'iridium se présentent sous forme pulvérulente.

De préférence, on utilise une poudre dont la granulométrie est inférieure à 300 nm. II est à noter que des composés dont la taille de particules est supérieure à cette valeur peuvent être utilisés dans le cadre de la présente invention. Cependant, pour des raisons de cinétique lors de la mise en oeuvre du procédé selon l'invention, il peut être préférable de broyer ceux-ci avant de les mettre en oeuvre.

Il a été trouvé d'une façon surprenante que les oxydes et/ou hydroxydes, hydratés ou non, d'iridium, mis en contact en phase liquide, avec l'acide iodhydrique, en présence d'un solvant, permettent d'obtenir une solution dans laquelle au moins 20 % et avantageusement au moins 40 % de l'iridium initial engagé se trouve sous une forme soluble.

Le fait que l'iridium engagé dans le procédé ne soit pas retrouvé en totalité dans la solution ne pose pas de problème particulier. En effet, dans la majeure partie des procédés industriels, le principal objectif n'est pas d'obtenir le rendement le plus élevé possible mais de trouver un compromis entre la productivité (rentabilité) et le rendement. Or dans ce cas précis, une solubilisation d'au moins 20 % et plus particulièrement au moins 40 % de l'iridium engagé représente un bon compromis, sachant que ce qui est resté sous une forme solide peut être recyclé lors d'une campagne de production ultérieure.

L'acide iodhydrique peut être mis en oeuvre sous la forme d'un gaz, sous la forme d'une solution, ou encore sous la forme d'un précurseur.

Comme précurseur susceptible de libérer l'acide iodhydrique, on peut mentionner à titre d'exemple, l'iode, les iodures d'alkyle en C₁-C₁₀, ou encore les iodures d'alcoyles en C₁-C₁₀.

Selon un mode particulier de réalisation de l'invention, l'acide iodhydrique est utilisé sous la forme d'une solution aqueuse. Bien que tous les degrés de dilution de l'acide conviennent à la mise en oeuvre du procédé, on préfère utiliser des solutions aqueuses présentant un titre en acide compris entre 40 et 70%.

La quantité d'acide iodhydrique mise en jeu dans le procédé selon l'invention varie dans de larges limites et doit être suffisante pour obtenir au moins une solution à base d'iridium telle que décrite ci-dessus. Ainsi, le procédé selon l'invention est mis en oeuvre de telle sorte que le nombre de moles d'acide, rapporté au nombre de moles d'iridium varie entre 1 et 100. Par les termes "quantité d'acide iodhydrique", on entend la quantité d'acide employé en tant que tel ou bien celle libérée par le précurseur si ce composé est mis en oeuvre.

Selon un mode préféré, le nombre de moles d'acide, rapporté au nombre de moles d'iridium présent est comprise entre 1 et 50.

Comme cela a été indiqué précédemment, le composé à base d'iridium est mis en contact avec l'acide iodhydrique ou un précurseur, en présence d'un solvant.

Tous les produits peuvent être utilisés dans la mesure où ils solubilisent, au moins partiellement, l'acide iodhydrique et le composé à base d'iridium. Toutefois, on met en oeuvre plus particulièrement, des solvants choisis parmi l'eau, les acides carboxyliques insaturés ou non, linéaires ou ramifiés, comprenant de 1 à 10 atomes de carbone, les esters d'acides carboxyliques insaturés ou non, linéaires ou ramifiés, comprenant 2 à 20 atomes de carbone, ou leur mélange.

De préférence, le choix du solvant est déterminé en fonction de l'application ultérieure de la solution obtenue. Ainsi, à titre d'exemple, on peut choisir l'acide acétique et/ou l'acétate de méthyle en cas de préparation d'acide acétique. On peut utiliser l'acide adipique et/ou de l'adipate de méthyle, ou encore l'acide penténoïque ou le penténoate de méthyle pour l'obtention de l'acide adipique.

Selon une première variante, le procédé selon l'invention est avantageusement mis en oeuvre sous air.

Une seconde variante du procédé consiste à effectuer la réaction en présence de monoxyde de carbone.

Une dernière variante du procédé selon l'invention consiste à effectuer la mise en contact du ou des composés iridiés avec l'acide iodhydrique, en présence d'hydrogène et/ou d'un gaz tel que l'azote ou les gaz rares, comme l'hélium ou encore l'argon.

Bien entendu, le procédé selon l'invention peut être mis en oeuvre en combinant les variantes précitées.

La mise en contact est réalisée dans un large domaine de pression. Ainsi, on peut effectuer la réaction à une pression de 1 à 200 bar.

La mise en contact proprement dite des réactifs peut être réalisée selon toutes méthodes connues de l'homme de l'art.

Ainsi, le composé à base d'iridium peut être introduit dans l'acide iodhydrique, sachant qu'une introduction inversée ou une mise en contact simultanée des deux réactifs est possible.

Par ailleurs, l'un et/ou l'autre de ces deux composés peuvent être mis en contact directement ou bien, chacun, sous la forme d'un mélange avec l'un ou plusieurs des solvants précités.

Dans le cas où le procédé est effectué selon l'une et/ou l'autre des trois mode de réalisation mentionnés, I'atmosphère de monoxyde de carbone, d'hydrogène, d'azote et/ou de gaz rare, peut être installée avant, pendant ou après la mise en contact des réactifs.

D'une façon classique, le procédé est réalisé sous agitation.

La durée de mise en contact n'est pas critique et l'homme du métier est à même de la fixer, selon qu'il privilégie la rentabilité du procédé ou encore une solubilisation maximale du composé iridié. A titre d'indication, cette durée varie entre une dizaine de minutes et une vingtaine d'heures.

Habituellement, la température à laquelle est mise en oeuvre le procédé est comprise entre 50 et 200°C.

La présente invention a pour second objet une solution à base d'iridium susceptible d'être obtenue en effectuant le procédé décrit auparavant. Par conséquent, tout ce qui a été explicité au sujet du procédé de préparation de la solution reste valable.

Enfin, l'invention a trait à l'utilisation de ladite solution à base d'iridium en tant que catalyseur.

Ainsi, la solution selon l'invention peut être utilisée telle quelle pour la réaction, comme c'est le cas pour les catalyses en phase homogène.

Elle peut de même être utilisée pour préparer un catalyseur solide (supporté ou non) en appliquant les méthodes classiques.

On peut ainsi envisager sécher la solution obtenue, en présence éventuellement d'un support convenable pour la réaction dans laquelle le catalyseur est destiné à être utilisé, de façon à obtenir des particules à base d'iridium. Il est par ailleurs possible d'imprégner ledit support, chacune des étapes précitées (séchage, imprégnation) étant ou non suivies de périodes de chauffage/frittage.

Cependant, selon un mode de réalisation préféré de l'invention, la solution est utilisée en tant que catalyseur, ou partie d'un système catalytique, pour effectuer des réactions en phase homogène.

La solution objet de la présente invention peut être mise directement en contact avec le mélange réactionnel ou bien être préalablement traitée pour la rendre parfaitement compatible avec ledit milieu réactionnel. Par traitement préalable, on entend notamment, ajuster les teneurs en certains des composés de la solution, ou compléter la composition de la solution en y ajoutant des constituants qui ne s'y trouvaient pas, à l'issue de son procédé de préparation, ou encore modifier l'atmosphère sous laquelle se trouve la solution.

La solution selon l'invention peut notamment être employée comme catalyseur de réactions de carbonylation, d'hydroformylation, d'isomérisation.

Cependant, la présente solution est plus particulièrement adaptée pour effectuer des réactions de carbonylation en présence de monoxyde de carbone, en phase liquide, en vue d'obtenir des acides carboxyliques et/ou des anhydrides d'acides carboxyliques. Il est à noter que ce type de réaction est bien connu et qu'il a fait l'objet de nombreux brevets et publications. Par conséquent, les conditions de réactions énoncées ci-dessous ne sont données qu'à titre général et ne peuvent être considérées comme limitatives.

Les réactifs mis en oeuvre pour ce genre de réaction sont choisis parmi les composés hydrocarbonés, linéaires, ramifiés ou cycliques, saturés ou non. On peut citer à titre illustratif, les alcènes ou alcynes, en C₂-C₁₀, les alcools en C₁-C₁₀, les dérivés halogénés desdits alcools, les éthers en C₂-C₂₀, les acides carboxyliques en C₃-C₁₀, les esters d'acides carboxyliques en C₂-C₂₀ de même que les dérivés halogénés desdits esters.

D'une façon classique, le système catalytique comprend d'une part la solution à base d'iridium et d'autre part un promoteur halogéné, choisi de préférence parmi les dérivés iodés comme les iodures d'alkyles et d'alcoyles.

Habituellement, le procédé consiste à faire réagir l'un des réactifs mentionnés ci-dessus en présence du système catalytique et de monoxyde de carbone, à une température variant de 50 à 300°C et sous une pression totale comprise entre 5 et 200 bar.

La réaction est en général effectuée en présence d'un solvant qui est plus particulièrement choisi parmi les produits et/ou réactifs mis en oeuvre dans la réaction.

Selon que l'objectif de la réaction est l'obtention de l'acide carboxylique (ou l'ester correspondant) ou l'anhydride d'acide carboxylique, la réaction est mise en oeuvre dans des conditions anhydres ou non. Ainsi, dans le premier cas, le milieu réactionnel comprend en général de l'eau alors que ce ne peut être le cas lors de l'obtention d'anhydride.

Une application préférée de la solution catalytique selon l'invention consiste à effectuer la carbonylation d'alcools, ou des dérivés halogénés correspondants, en acides carboxyliques.

Dans ce dernier cas, la réaction est menée de préférence en maintenant dans le milieu réactionnel des teneurs en eau, en promoteur halogéné comprises entre 0 exclu et 10 %, des teneurs en alcool et en ester, correspondant à l'alcool et à l'acide carboxylique formé, respectivement comprise entre 0 et 10 % et comprise entre 2 et 40 %, le reste étant constitué par ledit acide formé.

Des exemples concrets non limitatifs de l'invention vont maintenant être présentés.

### EXEMPLES

### EXEMPLE 1

Cet exemple illustre l'obtention d'une solution à partir de l'oxyde d'iridium Ir₂O₃ ,x H₂O.

Dans une ampoule en verre on introduit :
* 0,168 g d'oxyde d'iridium (STREM) ;
* 2,59 g d'acide iodhydrique en solution à 57 % dans l'eau
* 12 g d'acide acétique.

L'ampoule est ensuite placée dans un autoclave que l'on pressurise avec 5 bar de monoxyde de carbone.
On porte la température à 180°C puis on établit, une fois la température requise atteinte, une pression totale de 50 bar, au moyen de monoxyde de carbone.
La durée de la réaction est de 8 heures.

L'autoclave est ensuite dépressurisé puis le milieu réactionnel refroidi.

On obtient une solution de couleur rouge, que l'on décante.
On dose l'iridium dissous dans la phase liquide par spectroscopie à absorption atomique.
71 % de l'iridium engagé ont été solubilisés.

### EXEMPLE 2

Cet exemple illustre l'obtention d'une solution à partir de l'oxyde d'iridium non hydraté IrO₂.

On procède de la même façon que dans l'exemple 1 excepté le fait que la composition du mélange introduit dans l'ampoule est la suivante :
* 0,311 g d'oxyde d'iridium (STREM) ;
* 9 g d'acide iodhydrique en solution à 57 % dans l'eau ;
* 22 g d'acide acétique.

Après 17 heures de réaction, l'autoclave est dépressurisé et l'on obtient une solution de couleur rouge que l'on décante.
Le dosage par spectroscopie d'absorption indique que 40 % de l'iridium engagé se trouvent en solution.

### EXEMPLE 3

L'obtention d'une solution à partir de l'oxyde d'iridium hydraté IrO₂, x H₂O va maintenant être décrite.

La procédure suivie est identique à celle mise en oeuvre dans l'exemple 1 excepté le fait que la composition du mélange mise à réagir est la suivante :
* 0,437 g d'oxyde d'iridium hydraté (DEGUSSA) ;
* 9 g d'acide iodhydrique en solution à 57 % dans l'eau ;
* 22 g d'acide acétique.

La réaction a une durée de 8 heures.
Après dépressurisation de l'autoclave et refroidissement du milieu réactionnel, on obtient une solution de couleur rouge.
L'analyse par spectroscopie à absorption atomique indique que 100 % de l'iridium engagé se trouvent en solution.

### EXEMPLE 4

Cet exemple concerne l'obtention d'une solution à partir d'oxyde d'iridium hydraté IrO₂, xH₂O.

On introduit les composé suivants dans un ballon :
* 0,437 g d'oxyde d'iridium hydraté (DEGUSSA) ;
* 9 g d'acide iodhydrique en solution à 57 % dans l'eau ;
* 22 g d'acide acétique.

Le mélange est porté à 100 °C et mis à réagir pendant 10 minutes sous air.
Après refroidissement du mélange réactionnel, l'analyse par spectroscopie à absorption atomique de la solution résultante, de couleur rouge, indique que 100 % de l'iridium engagé se trouve en solution.

### EXEMPLE 6 COMPARATIF

Dans une ampoule, on introduit :
* 0,14 g d'oxyde d'iridium IrO₂ (STREM) ;
* 0,5 g d'iodure de méthyle ;
* 20 g de méthanol.
* 52 g d'acide acétique ;

L'ampoule est ensuite placée dans un autoclave que l'on pressurise avec 5 bar de monoxyde de carbone.
On porte la température à 195°C puis on établit, une fois la température requise atteinte, une pression totale de 70 bar, au moyen de monoxyde de carbone.
La durée de la réaction est de 3 heures.

A l'issue de la réaction, l'autoclave est dépressurisé puis le milieu réactionnel refroidi.

On obtient une solution incolore et un solide déposé au fond de l'ampoule.
Le dosage de l'iridium par spectroscopie à absorption atomique que la totalité de l'iridium engagé est restée sous forme solide.
Il est à noter par ailleurs qu'aucune carbonylation n'a été constatée dans ces conditions.

### EXEMPLE 7

Cet exemple a pour objet l'application de la solution catalytique obtenue dans l'exemple 1 pour la carbonylation du méthanol en acide acétique.
Cet essai est réalisé en discontinu.

Dans une ampoule en verre, on introduit :
* 1,85 g de la solution obtenue à l'exemple 1 ;
* 0,33 g d'eau ;
* 0,87 g de méthanol ;
* 0,33 g d'acétate de méthyle ;
* 0,77 g d'iodure de méthyle ;
* 11,6 g d'acide acétique.

L'ampoule est ensuite placée dans un autoclave que l'on pressurise avec 5 bar de monoxyde de carbone.
On porte la température à 185°C puis on établit, une fois la température requise atteinte, une pression totale de 30 bar, au moyen de monoxyde de carbone.

Dans ces conditions la vitesse de carbonylation du méthanol, obtenue par la mesure du débit de consommation du monoxyde de carbone, est de 4 mol/h.l. Il n'a pas été constaté l'existence d'une période d'initiation de la réaction (la vitesse mentionnée ci-dessus a été atteinte immédiatement).

### EXEMPLE 8

Cet exemple a pour objet l'application de la solution catalytique obtenue dans l'exemple 2 pour la carbonylation du méthanol en acide acétique.

On procède de la même manière que dans l'exemple 7, excepté le fait que la composition du mélange réactionnel est la suivante :
* 2,24 g de la solution obtenue à l'exemple 2 ;
* 0,38 g d'eau ;
* 1,3 g de méthanol ;
* 0,5 g d'acétate de méthyle ;
* 1,15 g d'iodure de méthyle ;
* 17,9 g d'acide acétique.

Dans ces conditions la vitesse de carbonylation du méthanol est de 4 mol/h.l.
Il n'a pas été constaté l'existence d'une période d'initiation de la réaction (la vitesse mentionnée ci-dessus a été atteinte immédiatement).

## Revendications

1. Procédé de préparation d'une solution à base d'iridium, caractérisé en ce que l'on met en contact, en phase liquide, au moins un oxyde ou un hydroxyde, hydraté ou non, d'iridium, ou leur mélange, en présence d'un solvant, avec de l'acide iodhydrique ou un composé susceptible de libérer de l'acide iodhydrique, dans une quantité telle que le nombre de moles d'acide iodhydrique, rapporté au nombre de moles d'iridium soit compris entre 1 et 100.

2. Procédé selon la revendication précédente, caractérisé en ce que l'on utilise un nombre de moles d'acide iodhydrique, rapporté au nombre de moles d'iridium, compris entre 1 et 50.

3. Procédé selon l'une quelconque des revendications précédentes, caractérisé en ce que l'on utilise un solvant choisi parmi l'eau, les acides carboxyliques insaturés ou non, linéaires ou ramifés, comprenant de 1 à 10 atomes de carbone, les esters d'acides carboxyliques insaturés ou non, linéaires ou ramifés, comprenant 2 à 20 atomes de carbone, ou leur mélange.

4. Procédé selon l'une quelconque des revendications précédentes, caractérisé en ce que l'on effectue la mise en contact sous air.

5. Procédé selon l'une quelconque des revendications précédentes, caractérisé en ce que l'on effectue la mise en contact en présence de monoxyde de carbone.

6. Procédé de préparation selon l'une quelconque des revendications précédentes, caractérisé en ce que l'on effectue la mise en contact en présence d'hydrogène, d'azote et/ou d'un gaz rare comme l'argon ou l'hélium.

7. Procédé de préparation selon l'une quelconque des revendications précédentes, caractérisé en ce que l'on effectue la mise en contact à une pression entre 1 et 200 bar.

8. Solution à base d'iridium susceptible d'être obtenue en mettant en oeuvre le procédé selon l'une des revendications 1 à 7 et dans laquelle au moins 20 % de l'iridium par rapport à l'iridium engagé se trouve sous forme soluble.

9. Utilisation de la solution selon la revendication 8 en tant que catalyseur ou pour la préparation d'un catalyseur.

10. Utilisation selon la revendication 9, caractérisée en ce que ladite solution est utilisée comme catalyseur ou pour la préparation d'un catalyseur dans un procédé de carbonylation de composés hydrocarbonés, linéaires, ramifiés ou cycliques, saturés ou non, tels que les alcènes, les alcynes en C₂-C₁₀, les alcools en C₁-C₁₀, les dérivés halogénés desdits alcools, les éthers en C₂-C₂₀, les acides carboxyliques en C₃-C₁₀, les esters d'acides carboxyliques en C₂-C₂₀ de même que les dérivés halogénés desdits esters.

11. Utilisation selon la revendication 9, caractérisée en ce que ladite solution est utilisée pour catalyser une réaction de carbonylation d'un alcool en présence de monoxyde de carbone et pour donner l'acide carboxylique correspondant, en maintenant dans le mélange réactionnel des teneurs de 0 exclu à 10 % en eau et en promoteur halogéné, des teneurs de 0 à 10 % en alcool et de 2 à 40 % en ester correspondant à l'acide et à l'alcool ; le reste étant constitué par l'acide carboxylique.

## Claims

1. A process for the preparation of an iridium-based solution, characterised in that at least one hydrated or non-hydrated iridium oxide or hydroxide, or a mixture thereof, is placed in contact, in liquid phase and in the presence of a solvent, with hydroiodic acid or a compound capable of releasing hydroiodic acid, in an amount such that the number of moles of hydroiodic acid, with respect to the number of moles of iridium be between 1 and 100.

2. The process according to the preceding claim, characterised in that a number of moles of hydroiodic acid with respect to the number of moles of iridium between 1 and 50 is used.

3. The process according to any one of the preceding claims, characterised in that a solvent selected from water, linear or branched, saturated or unsaturated carboxylic acids having 1 to 10 carbon atoms, linear or branched, saturated or unsaturated carboxylic acid esters having 2 to 20 carbon atoms, or a mixture thereof, is used.

4. The process according to any one of the preceding claims, characterised in that the placing in contact is carried out under air.

5. The process according to any one of the preceding claims, characterised in that the placing in contact is carried out in the presence of carbon monoxide.

6. The process of preparation according to any one of the preceding claims, characterised in that the placing in contact is carried out in the presence of hydrogen, nitrogen and/or a rare gas such as argon or helium.

7. The process of preparation according to any one of the preceding claims, characterised in that the placing in contact is carried out at a pressure between 1 and 200 bar.

8. An iridium-based solution obtainable by carrying out the process according to one of claims 1 to 7 and in which at least 20% of the iridium with respect to the iridium engaged is found in a soluble form.

9. Use of the solution according to claim 8 as a catalyst or for the preparation of a catalyst.

10. The use according to claim 9, characterised in that said solution is used as a catalyst, or for the preparation of a catalyst, in a process of carbonylation of saturated or unsaturated linear, branched or cyclic hydrocarbon compounds such as C₂-C₁₀ alkenes and alkynes, C₁-C₁₀ alcohols, halogenated derivatives of said alcohols, C₂-C₂₀ ethers, C₃-C₁₀ carboxylic acids, C₂-C₂₀ carboxylic acid esters, as well as halogenated derivatives of said esters.

11. The use according to claim 9, characterised in that said solution is used for catalysing a reaction of carbonylation of an alcohol in the presence of carbon monoxide and for giving the corresponding carboxylic acid, in maintaining the water content and the halogenated promoter content of 0 (excluded) to 10%, the alcohol content of 0 to 10%, and the content of the ester corresponding to the acid and the alcohol of 2 to 40%, in the reaction medium; the remainder being constituted by carboxylic acid.

## Patentansprüche

1. Verfahren zur Herstellung einer Lösung auf Iridium-Basis, dadurch gekennzeichnet, daß man in flüssiger Phase mindestens ein hydratisiertes oder nicht-hydratisiertes Oxid oder Hydroxid von Iridium oder eine Mischung davon in Gegenwart eines Lösungsmittels mit Iodwasserstoffsaure oder einer Verbindung, die Iodwasserstoffsäure freisetzen kann, in einer solchen Menge in Kontakt bringt, daß die Anzahl der Mole Iodwasserstoffsäure, bezogen auf die Anzahl der Mole Iridium, zwischen 1 und 100 liegt.

2. Verfahren nach dem vorhergehenden Anspruch, dadurch gekennzeichnet, daß man eine Anzahl von Molen Iodwasserstoffsäure, bezogen auf die Anzahl der Mole Iridium, verwendet, die zwischen 1 und 50 liegt.

3. Verfahren nach einem der vorhergehenden Ansprüche, dadurch gekennzeichnet, daß man ein Lösungsmittel verwendet, das ausgewählt wird aus der Gruppe Wasser, der ungesättigten oder gesättigten, linearen oder verzweigten Carbonsäuren mit 1 bis 10 Kohlenstoffatomen, der ungesättigten oder gesättigten, linearen oder verzweigten Carbonsäureester mit 2 bis 20 Kohlenstoffatomen oder einer Mischung davon.

4. Verfahren nach einem der vorhergehenden Ansprüche, dadurch gekennzeichnet, daß man das Inkontaktbringen an der Luft durchführt.

5. Verfahren nach einem der vorhergehenden Ansprüche, dadurch gekennzeichnet, daß man das Inkontaktbringen in Gegenwart von Kohlenmonoxid durchführt.

6. Verfahren nach einem der vorhergehenden Ansprüche, dadurch gekennzeichnet, daß man das Inkontaktbringen in Gegenwart von Wasserstoff, Stickstoff und/oder eines Edelgases wie Argon oder Helium durchführt.

7. Verfahren nach einem der vorhergehenden Ansprüche, dadurch gekennzeichnet, daß man das Inkontaktbringen bei einem Druck zwischen 1 und 200 bar durchführt.

8. Lösung auf Iridium-Basis, wie sie unter Anwendung des Verfahrens nach einem der Ansprüche 1 bis 7 erhalten werden kann, in der mindestens 20 % Iridium, bezogen auf das gebundene Iridium, in löslicher Form vorliegen.

9. Verwendung der Lösung nach Anspruch 8 als Katalysator oder zur Herstellung eines Katalysators.

10. Verwendung nach Anspruch 9, dadurch gekennzeichnet, daß die genannte Lösung als Katalysator oder zur Herstellung eines Katalysators in einem Verfahren zur Carbonylierung von linearen, verzweigten oder cyclischen, gesättigten oder ungesättigten Kohlenwasserstoff-Verbindungen, wie Alkenen, C₂-C₁₀-Alkinen, C₁-C₁₀-Alkoholen, hydrierten Derivaten der genannten Alkohole, C₂-C₂₀-Ethern, C₃-C₁₀-Carbonsäuren, C₂-C₂₀-Carbonsäureestern sowie halogenierten Derivaten der genannten Ester verwendet wird.

11. Verwendung nach Anspruch 9, dadurch gekennzeichnet, daß die genannte Lösung zum Katalysieren einer Reaktion zur Carbonylierung eines Alkohols in Gegenwart von Kohlenmonoxid und zur Herstellung der entsprechenden Carbonsäure verwendet wird, wobei man in der Reaktionsmischung Gehalte an Wasser und an halogeniertem Promotor von 0 (der Wert 0 ist ausgeschlossen) bis 10 %, Alkohol-Gehalte von 0 bis 10 % und Gehalte an einem der Säure und dem Alkohol entsprechenden Ester von 2 bis 40 % aufrechterhält, wobei der Rest aus der Carbonsäure besteht.
